# EUROPÄISCHE PATENTANMELDUNG

(11) **EP 4 850 710 A1**
(43) Veröffentlichungstag der Anmeldung: **09.12.2026**
(21) Anmeldenummer: 26175595.3
(22) Anmeldetag: 30.04.2026
(51) Int. Cl.: A61M 1/00, A61F 9/007, A61M 3/02

(54) **CHIRURGISCHE KONSOLE UND SYSTEM FÜR OPHTHALMOLOGISCHE EINGRIFFE**

(30) Priorität: 03.06.2025 DE 102025121615
(71) Anmelder: Geuder AG, 69126 Heidelberg (DE)
(72) Erfinder: STEGMAIER, Lukas, 69226 Nußloch (DE); REUTER, Wolfgang, 61231 Bad Nauheim (DE)
(74) Vertreter: Ullrich & Naumann PartG mbB

(57) **Zusammenfassung**

Die Erfindung betrifft eine Chirurgische Konsole für ophthalmologische Eingriffe, umfassend ein Gehäuse mit darin befindlichen Funktionseinheiten, u.a. zur Aspiration und Irrigation, sowie mit Bedien- und Anzeigeelementen und Anschlüssen für ophthalmologische Geräte, wobei eine Aufnahme für eine ophthalmologische Kassette vorgesehen ist, wobei zur Förderung von Flüssigkeit in der Kassette mindestens eine Pumpe mit zumindest einem Pumpenelement vorgesehen ist, wobei die Pumpe derart ausgebildet ist, dass eine Medientrennung zwischen der Pumpe und der Flüssigkeit in der Kassette erfolgt, insbesondere wobei die Pumpe als eine über einen Rotor drehangetriebene Rollenpumpe ausgebildet ist, wobei eine Federeinrichtung angeordnet ist, die dazu ausgebildet ist, die Kassette sowie die Pumpe und/oder das Pumpenelement mit einer definierten Kraft zueinander und/oder gegeneinander zu drücken.

## Beschreibung

Die Erfindung betrifft eine Chirurgische Konsole für ophthalmologische Eingriffe, umfassend ein Gehäuse mit darin befindlichen Funktionseinheiten, u.a. zur Aspiration und Irrigation, sowie mit Bedien- und Anzeigeelementen und Anschlüssen für ophthalmologische Geräte, wobei eine Aufnahme für eine ophthalmologische Kassette vorgesehen ist, wobei zur Förderung von Flüssigkeit in der Kassette mindestens eine über einen Rotor drehangetriebene Pumpe vorgesehen ist, wobei die Pumpe derart ausgebildet ist, dass eine Medientrennung zwischen der Pumpe und der Flüssigkeit in der Kassette erfolgt.

Die Erfindung betrifft weiter ein chirurgisches System umfassend eine chirurgische Konsole sowie eine ophthalmologische Kassette.

Chirurgische Konsolen der in Rede stehenden Art sind bereits seit Jahren aus der Praxis bekannt. Diese dienen zur Durchführung von ophthalmologischen Operationen, bspw. einer Phakoemulsifikation. Dabei wird eine durch einen grauen Star getrübte Linse anhand einer mit Ultraschall beaufschlagten Nadel zertrümmert. Die Linsentrümmer werden durch die Nadel hindurch abgesaugt. Um ein Kollabieren sowie eine Überhitzung des Auges zu vermeiden, wird dem Auge während des Eingriffs eine Irrigationsflüssigkeit zugeführt. Somit ist das chirurgische Handgerät durch die Konsole während des Eingriffs mit Ultraschallleistung, mit Irrigations-flüssigkeit sowie mit einem Unterdruck zu versorgen.

Die chirurgischen Konsolen umfassen einen Schacht, in den eine Kassette einsetzbar ist. Innerhalb der Kassette ist ein Schlauchsystem angeordnet, durch das ein Irrigations- und/oder Aspirationsmedium förderbar ist. Kassette und Konsole bilden dabei ein chirurgisches System. Dabei wird das Irrigations- und/oder Aspirations-medium durch eine Rollenpumpe gefördert, die das Irrigations- und/oder Aspirationsmedium innerhalb des Schlauchsystems der Kassette walkt. Die Rollenpumpe ist dabei derart innerhalb der Konsole angeordnet, dass die Kassette in den Schacht eingesteckt und mit der Rollenpumpe in Wirkverbindung gebracht werden kann. Das heißt durch das Einstecken der Kassette in den Schacht entsteht die Wirkverbindung zwischen Rollenpumpe und Kassette.

Die Rollenpumpe walkt den Schlauch in der Kassette, um das Medium zu fördern. Dabei wird der Schlauch bedingt durch den Anpressdruck der Rollenpumpe zusammengequetscht, sodass das Medium innerhalb des Schlauchs gefördert wird. Bei einem Stromausfall verschließt die Rollenpumpe den Schlauch, sodass kein Medium mehr gefördert werden kann. Deswegen ist es bekannt geworden, zusätzliche Ventile bereitzustellen, die im Falle eines Defekts der Rollenpumpe einen Fluidkanal zwischen einem Irrigationsbehälter und dem Auge öffnen. Hierdurch kann gewährleistet werden, dass immer genügend Irrigationsflüssigkeit für das Auge vorhanden ist, um ein Zusammenbrechen des Kapselsacks zu verhindern.

Nachteilig an dieser Anordnung ist, dass der Aufbau komplex und kostenintensiv ist, da die Rollenpumpe und der Schlauch geringe Toleranzen aufweisen müssen, um einen definierten Volumenstrom zu gewährleisten.

Der vorliegenden Erfindung liegt daher die Aufgabe zugrunde, eine chirurgische Konsole und ein chirurgisches System der eingangs genannten Art derart auszugestalten und weiterzubilden, dass eine möglichst einfache Bauart und eine kostengünstige Herstellung ermöglicht wird, bei der Toleranzen der Komponenten ausgeglichen und/oder kompensiert werden können.

Erfindungsgemäß wird die voranstehende Aufgabe durch die Merkmale des Anspruches 1 gelöst. Danach ist eine chirurgische Konsole für ophthalmologische Eingriffe, umfassend ein Gehäuse mit darin befindlichen Funktionseinheiten, u.a. zur Aspiration und Irrigation, sowie mit Bedien- und Anzeigeelementen und Anschlüssen für ophthalmologische Geräte, wobei eine Aufnahme für eine ophthalmologische Kassette vorgesehen ist, wobei zur Förderung von Flüssigkeit in der Kassette mindestens eine Pumpe vorgesehen ist, wobei die Pumpe derart ausgebildet ist, dass eine Medientrennung zwischen der Pumpe und der Flüssigkeit in der Kassette erfolgt, insbesondere wobei die Pumpe als eine über einen Rotor drehangetriebene Rollenpumpe ausgebildet ist, dadurch gekennzeichnet, dass eine Federeinrichtung angeordnet ist, die dazu ausgebildet ist, die Kassette sowie die Pumpe und/oder das Pumpenelement mit einer definierten Kraft zueinander und/oder gegeneinander zu drücken.

Die voranstehende Aufgabe wird weiter durch die Merkmale des Anspruchs 9 gelöst. Danach ist ein chirurgisches System vorgesehen, umfassend eine chirurgische Konsole gemäß einem der Ansprüche 1 bis 8 sowie eine ophthalmologische Kassette, die in der Aufnahme der chirurgischen Konsole vorgesehen ist, wobei die Kassette dazu ausgebildet ist, mit der Pumpe der chirurgischen Konsole in Wirkverbindung zu treten, wenn die Kassette in der Aufnahme angeordnet ist.

Der Begriff "dazu ausgebildet, die Anordnung der Pumpe und/oder eines Pumpenelements sowie der Kassette relativ zueinander zu verändern" bedeutet in der Beschreibung, vorzugsweise in den Ansprüchen, dass die relative Position der Pumpe und der Kassette zueinander durch die Bewegungseinrichtung veränderbar ist und/oder dass zumindest die Anordnung eines Pumpenelements der Pumpe und der Kassette relativ zueinander änderbar ist. Insbesondere kann beispielsweise die Pumpe in eine erste Position und eine zweite Position verbracht werden, wohingegen die Position der Kassette konstant bleibt. Es ist ebenfalls denkbar, dass lediglich die Position der Kassette verändert wird, jedoch die Position der Pumpe konstant bleibt. Außerdem ist es denkbar, dass sowohl die Kassette als auch die Pumpe in ihrer Position verändert werden. Statt einer Bewegung der Pumpe ist es auch denkbar, dass lediglich ein Pumpenelement in seiner Position verändert wird. Das Pumpenelement ist ein Element der Pumpe, beispielsweise kann das Pumpenelement eine Rolle einer Rollenpumpe sein.

Der Begriff "Pumpenelement" ist im weitesten Sinne zu verstehen und bezieht sich in der Beschreibung, vorzugsweise in den Ansprüchen, auf ein Element der Pumpe, insbesondere ein Element der Pumpe, das direkt zur Förderung von Flüssigkeit genutzt wird.

Der Begriff "zueinander zu drücken" ist im weitesten Sinne zu verstehen und bezieht sich in der Beschreibung, vorzugsweise in den Ansprüchen, darauf, dass die Pumpe und/oder ein Pumpenelement der Pumpe sowie die Kassette relativ zueinander hin gedrückt werden. Beispielsweise kann die Kassette festgelegt sein und die Pumpe wird auf die Kassette gedrückt. Es ist ebenfalls denkbar, dass die Pumpe festgelegt ist und die Kassette auf die Pumpe gedrückt wird. Außerdem ist es denkbar, dass die Pumpe festgelegt ist, jedoch ein Pumpenelement der Pumpe in Richtung der Kassette gedrückt wird. Beispielsweise kann das Pumpenelement eine Rolle sein, die durch einen Federmechanismus auf den Schlauch gedrückt wird.

In erfindungsgemäßer Weise ist zunächst erkannt worden, dass durch das Vorsehen einer Federeinrichtung an der Pumpe die Kraft, mit der die Pumpe auf die Kassette, insbesondere das Schlauchsystem der Kassette, einwirkt, konstant gehalten werden kann, unabhängig von den Toleranzen des Schlauchsystems, der Kassette und/oder der Pumpe. Wenn beispielsweise durch entsprechende Toleranzen ein größerer Abstand zwischen der Pumpe und dem Schlauchsystem existiert, kann das Federsystem dennoch einen konstanten Anpressdruck zwischen Pumpe und Schlauchsystem erzeugen, da das Federsystem kraftgesteuert und nicht weggesteuert ist. Insbesondere kann das Federsystem dazu ausgebildet sein, eine konstante Kraft zu erzeugen. Des Weiteren kann das Federsystem dazu ausgebildet sein, die Pumpe und/oder ein Pumpenelement der Pumpe in Richtung der Kassette und/oder des Schlauchsystems zu drücken. Es ist ebenfalls denkbar, dass das Federsystem dazu ausgebildet ist, die Kassette in Richtung der Pumpe zu drücken. Somit kann eine relative Kraft zwischen der Kassette und der Pumpe zueinander gewährleistet werden. Durch einen konstanten Anpressdruck kann ein konstant genauer geförderter Volumenstrom der Pumpe erzeugt werden. Darüber hinaus ist ein Vorteil, dass Leckagen aufgrund von mangelndem Anpressdruck der Pumpe verhindert werden können.

Obwohl allgemein auf beliebige Pumpen anwendbar, die eine Medientrennung zwischen der Pumpe und der in der Kassette befindlichen Flüssigkeit ermöglicht, wird die nachfolgende Erfindung in Bezug auf Rollenpumpen beschrieben. Eine Rollenpumpe, auch Peristaltik-Pumpe genannt, umfasst mehrere Rollen, die an einem Rotierenden Kernstück angeordnet sind. Durch Drehen des Kernstücks werden die Rollen entlang einer Kreisbahn bewegt. Wenn nun ein Schlauch entlang eines bogenförmigen Anschlags angeordnet wird, und die Rollenpumpe an dem Anschlag angeordnet wird, kann durch die Bewegung der Rollen der Schlauch gequetscht werden und so eine Flüssigkeit in dem Schlauch gepumpt werden. Dabei treten die Rollen der Rollenpumpe und auch die Pumpe selbst, niemals in direkten Kontakt mit der Flüssigkeit, sondern der Kontakt erfolgt ausschließlich indirekt über den Schlauch. So wird eine Medientrennung zwischen der in dem Schlauch und somit in der Kassette befindlichen Flüssigkeit und der Pumpe ermöglicht.

Die Aufnahme für die ophthalmologische Kassette kann insbesondere ein Schacht sein, in den die ophthalmologische Kassette einsetzbar und/oder einführbar ist.

Die nachfolgend benannten vorteilhaften Weiterbildungen gelten sowohl für die Konsole als auch für das chirurgische System.

Gemäß einer vorteilhaften Weiterbildung der Erfindung ist eine Bewegungseinrichtung vorgesehen, die dazu ausgebildet ist, die Anordnung der Pumpe und/oder eines Pumpenelements sowie der Kassette relativ zueinander zu verändern, wobei die Pumpe und/oder das Pumpenelement sowie die Kassette in einer ersten Anordnung relativ zueinander anordenbar sind, in der die Pumpe ein Strömen der Flüssigkeit innerhalb der Kassette verhindern kann und wobei die Pumpe und/oder das Pumpenelement sowie die Kassette in einer zweiten Anordnung relativ zueinander anordenbar sind, in der die Pumpe ein Strömen der Flüssigkeit innerhalb der Kassette ermöglicht. Hierdurch kann das Pumpenelement eine Ventilfunktion ausüben, da durch das Pumpenelement der Fluidweg innerhalb der Kassette geöffnet und/oder verschlossen werden kann.

Bei der Federeinrichtung kann es sich insbesondere um eine Feder und/oder ein pneumatisches Element handeln. Beispielsweise kann ein pneumatisch betriebener Kolben vorgesehen sein, der die Pumpe gegen die Kassette drückt. Die Feder und/oder das pneumatische Element kann an der Pumpe angeordnet sein und/oder zwischen der Pumpe und der Kassette und/oder an der Kassette. Die Feder kann als Zugfeder oder als Druckfeder ausgebildet sein und beispielsweise eine mechanische Feder sein. Die Federeinrichtung kann eine im Wesentlichen konstante Kraft aufbringen, die die Pumpe und/oder das Pumpenelement sowie die Kassette aneinanderdrückt. Somit werden die Kassette und die Pumpe immer mit einer konstanten Kraft aufeinandergepresst, unabhängig von den Toleranzen der Kassette, des Schlauchsystems in der Kassette oder der Pumpe. Da der Anpressdruck den Volumenstrom bestimmt, mit dem beispielsweise eine Rollenpumpe Fluid in der Kassette fördern kann, kann auf diese Weise ein konstanter Volumenstrom bereitgestellt werden. Ein Vorteil hiervon ist, dass Toleranzen des chirurgischen Systems, das heißt insbesondere Fertigungstoleranzen der Pumpe, der Kassette und/oder der der Konsole, ausgeglichen werden können.

In einer vorteilhaften Ausgestaltung der Erfindung ist ein Behälter mit Irrigations-flüssigkeit vorgesehen, wobei ein Fluidweg zwischen dem Behälter mit Irrigations-flüssigkeit und den Anschlüssen für die ophthalmischen Geräte frei von Ventilen ausgebildet ist. Mit anderen Worten ist direkter Fluidweg zwischen dem Irrigationsbehälter und den Anschlüssen für das Handgerät vorgesehen, sodass im Falle einer Fehlfunktion der Rollenpump Flüssigkeit direkt aus dem Irrigationsbehälter zu dem Handgerät geleitet werden kann. Ein Vorteil hiervon ist, dass die Gefahr eines Zusammenbrechens des Kapselsacks verringert werden kann.

In ähnlicher Weise kann ein Behälter zur Aufnahme von Aspirationsflüssigkeit vorgesehen sein, wobei ein Fluidweg zwischen dem Behälter für Aspirationsflüssigkeit und den Anschlüssen für die ophthalmischen Geräte frei von Ventilen ausgebildet ist. Durch die Pumpe kann ebenfalls der Aspirationsfluidweg im Sinne einer Ventilwirkung gesteuert werden, sodass auch der Aspirationsweg innerhalb der Konsole frei von Ventilen ausgebildet sein kann. Insbesondere kann eine zusätzliche Pumpe oder dieselbe Pumpe für den Irrigationsweg zum Verschließen des Aspirationswegs genutzt werden.

Gemäß einer vorteilhaften Weiterbildung der Erfindung ist eine weitere Federeinrichtung angeordnet ist, wobei die weitere Federeinrichtung dazu ausgebildet ist, die Kassette mit einer definierten Einzugskraft in die Aufnahme zu ziehen. Bei der weiteren Federeinrichtung kann es sich um eine mechanische Feder oder einen pneumatischen Kolben handeln. Die weitere Federeinrichtung ist dazu ausgebildet, die Kassette mit einer definierten Einzugskraft in den Schacht zu ziehen, mit anderen Worten die Kassette in der Wirkposition zu halten. Hierdurch erfolgt die Bewegung der Kassette relativ zu der Pumpe kraftgesteuert und nicht weggesteuert. Ein Vorteil hiervon ist, dass die benötigten Toleranzen des Schachts und der Kassette geringer sein können, insbesondere weil die Bewegung der Kassette nicht um eine vordefinierte Bewegung erfolgt.

Insbesondere kann dabei eine mechanische Feder angeordnet sein, die die Pumpe und/oder die Kassette in die zweite Position drückt, also in diejenige Position in der Flüssigkeit frei durch die Kassette strömen kann. Durch die weitere Federeinrichtung, insbesondere in Form eines pneumatischen Kolbens, kann die Federkraft der mechanischen Feder überwunden werden und so die Pumpe und/oder die Kassette in die erste Position gedrückt werden. In dieser Position wird ein Strömen von Flüssigkeit in der Kassette verhindert, solange die Pumpe nicht betätigt wird. Gleichzeitig kann durch ein Betätigen der Pumpe, das heißt insbesondere durch Drehen der Rollen der Rollenpumpe, die Flüssigkeit aktiv gefördert werden. Insbesondere ist die Bewegungseinrichtung die weitere Federeinrichtung.

Dabei ist es insbesondere von Vorteil, wenn die weitere Federeinrichtung einen Hebel umfasst, der zwischen Pumpe und Federeinrichtung angeordnet ist. Durch den Hebel kann eine Bewegung der Pumpe genauer gesteuert werden.

Vorteilhafterweise umfasst die weitere Federeinrichtung ein pneumatisches Element, wobei das pneumatische Element dazu ausgebildet ist, die Einzugskraft zu steuern. Die Einzugskraft bestimmt die Position der Kassette relativ zu der Pumpe in dem Schacht. Durch ein pneumatisches Element kann diese Einzugskraft gesteuert werden, sodass der Anpressdruck und/oder die Position der Kassette gesteuert werden kann. Ein Vorteil hiervon ist, dass die Kassette nachgedrückt werden kann und dass die Verdrängung durch die Pumpe an bestimmten Punkten minimiert werden kann.

Gemäß einer vorteilhaften Ausgestaltung der Erfindung ist durch die Pumpe eine Flüssigkeit in der Kassette förderbar, wenn sich die Pumpe in der ersten Position befindet. Somit kann die Pumpe in der ersten Position sich drehen, sodass hierdurch direkt das Schlauchsystem in der Kassette gewalkt werden kann. Ein Vorteil hiervon ist, dass lediglich zwei Positionen der Pumpe definiert werden müssen, da die Pumpe in der ersten Position zwei Funktionen übernehmen kann, eine Ventilfunktion und eine Pumpfunktion.

Gemäß einer vorteilhaften Ausgestaltung der Erfindung ist ein Fluidweg innerhalb der Kassette verschließbar, wenn die Pumpe in der ersten Position angeordnet ist, wobei die Pumpe eine Flüssigkeit in dem Fluidweg fördern kann, wenn die Pumpe in der ersten Position angeordnet ist und wobei eine Flüssigkeit durch den Fluidweg strömen kann, wenn die Pumpe in der zweiten Position angeordnet ist. Mit anderen Worten kann die Pumpe in der ersten Position eine Ventilfunktion aufweisen, sodass die Pumpe ein Strömen von Flüssigkeit in der Kassette verhindern kann. In der zweiten Position hingegen ist das Ventil geöffnet und Flüssigkeit kann frei durch die Kassette fließen. Um dies zu ermöglich kann die Pumpe um einen bestimmten Weg in Richtung des Schlauchsystems innerhalb der Kassette verfahren werden. Insbesondere kann der minimale Weg, um den die Pumpe verfahrbar ist, dem Durchmesser des Schlauchinneren entsprechen. So kann gewährleistet werden, dass der Schlauch vollständig von der Pumpe abgeklemmt werden kann.

Es gibt nun verschiedene Möglichkeiten, die Lehre der vorliegenden Erfindung in vorteilhafter Weise auszugestalten und weiterzubilden. Dazu ist einerseits auf die dem Anspruch 1 nachgeordneten Ansprüche und andererseits auf die nachfolgende Erläuterung bevorzugter Ausführungsbeispiele der Erfindung anhand der Zeichnung zu verweisen. In Verbindung mit der Erläuterung der bevorzugten Ausführungsbeispiele der Erfindung anhand der Zeichnung werden auch im Allgemeinen bevorzugte Ausgestaltungen und Weiterbildungen der Lehre erläutert. In der Zeichnung zeigen
- Fig. 1: eine Kassette aus dem Stand der Technik.
- Fig. 2: eine Konsole gemäß einer Ausführungsform der vorliegenden Erfindung.
- Fig. 3: die beiden Positionen der Rollenpumpe.
- Fig. 4: eine Kassette gemäß einer Ausführungsform der vorliegenden Erfindung
- Fig. 5: ein chirurgisches System gemäß einer Ausführungsform der vorliegenden Erfindung

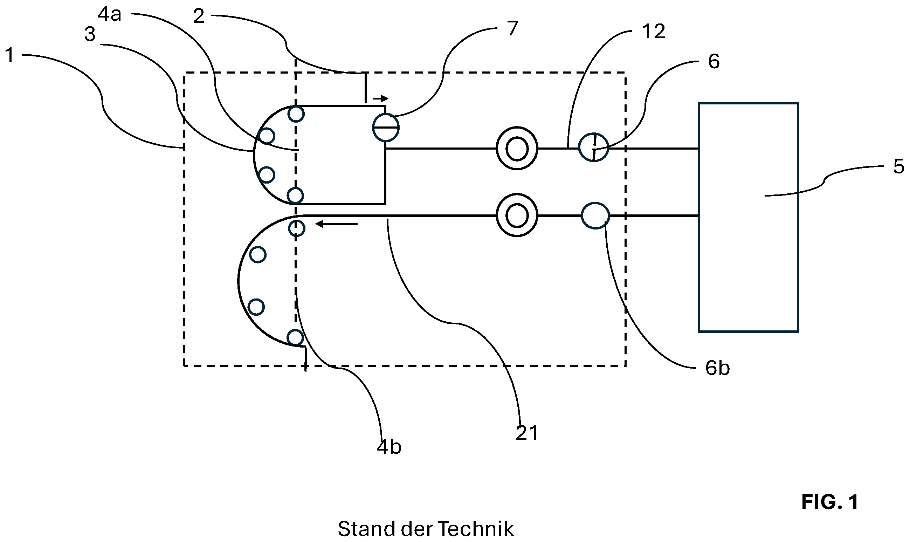

Fig. 1 zeigt eine Kassette, wie sie aus dem Stand der Technik bekannt ist.

Die Kassette 1 umfasst einen Einlass 2, durch den Irrigationsflüssigkeit in die Kassette 1 strömen kann. Die Irrigationsflüssigkeit strömt anschließend durch einen Bogen 3, an dem eine Pumpe 4a, insbesondere in Form einer Rollenpumpe 4a angeordnet ist. Die Rollenpumpe 4a verdrängt die Irrigationsflüssigkeit, sodass die Irrigationsflüssigkeit anschließend entlang eines Irrigationsfluidwegs 12 zu einer Patientenschnittstelle 5 gefördert werden kann, die Anschlüsse für Handstücke, z.B. ein Phacoinstrument umfasst.

Zwischen der Patientenschnittstelle 5 und der Rollenpumpe 4 ist ein Sperrventil 6 angeordnet, mit dem der Strom der Irrigationsflüssigkeit bei Bedarf unterbrochen werden kann. Zusätzlich ist ein Sicherheitsventil 7 vorgesehen, das einen alternativen Strömungspfad zwischen dem Einlass 2 und der Patientenschnittstelle 5 öffnen und/oder freigeben kann. Im Falle eines Stromausfalls kann das Sicherheitsventil 7 geöffnet werden, sodass der alternative Strömungspfad geöffnet wird. Auf diese Weise kann sichergestellt werden, dass trotz Stromausfalls Irrigationsflüssigkeit zu der Patientenschnittstelle 5 gelangen kann.

Ausgehend von der Patientenschnittstelle 5 kann Aspirationsflüssigkeit durch eine zweite Rollenpumpe 4b angesaugt und entlang eines Aspirationsfluidweges 21 zu einem Auslass 8 gefördert werden. Dieser Aspirationsfluidweg 21 kann ebenfalls durch ein Sperrventil 6b unterbrochen werden, um bei Bedarf den Druck in dem Auge des Patienten zu regulieren.

Diese Anordnung ist aufwendig, da sie mehrere Ventile 6, 6b und 7 benötigt.

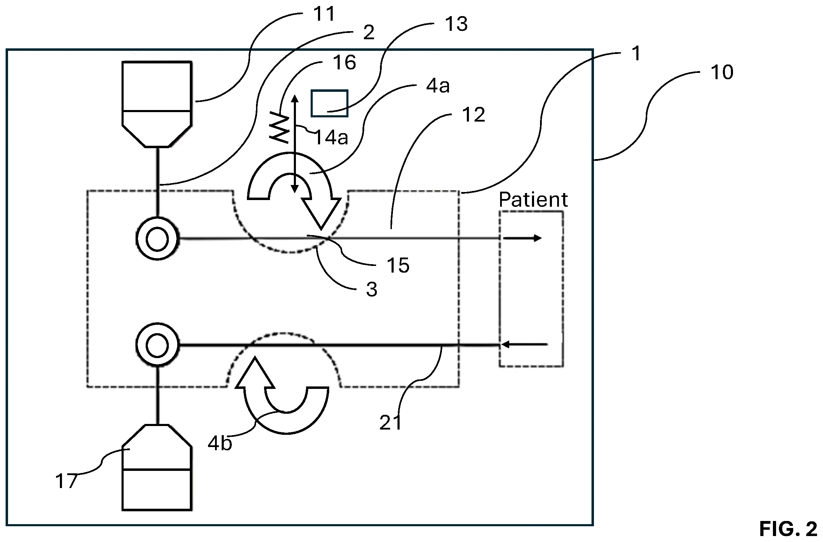

Figur 2 zeigt eine Konsole gemäß einer Ausführungsform der vorliegenden Erfindung.

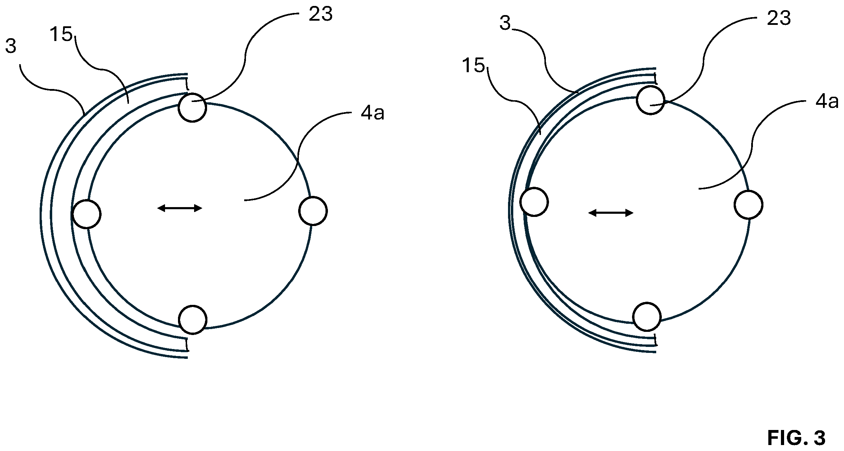

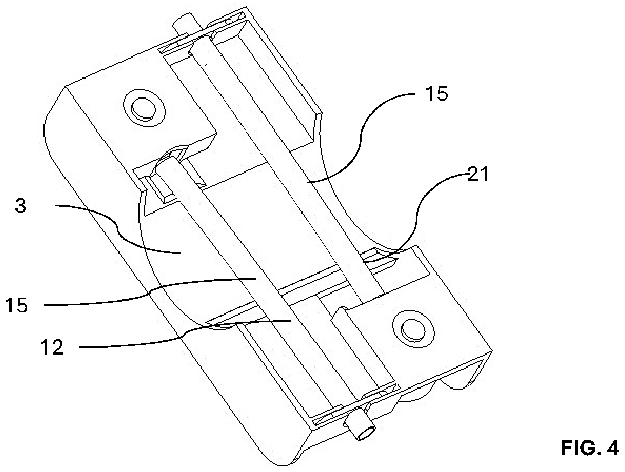

Die Konsole 10 umfasst eine Kassette 1, die in einem Schacht (nicht gezeigt) der Konsole 10 angeordnet ist. Eine solche Kassette 1 ist in Figur 4 näher gezeigt. Ausgehend von einem Irrigationsbehälter 11 kann Irrigationsflüssigkeit durch einen Einlass 2 in die Kassette 1 strömen. Die Kassette 1 umfasst einen Bogen 3, in den eine Pumpe 4a der Konsole 10 eingreifen kann. Die Pumpe 4a ist derart ausgebildet, dass eine Medientrennung zwischen der Pumpe 4a und der in der Kassette 1 befindlichen Flüssigkeit erfolgt. Das heißt, dass die Flüssigkeit in der Kassette 1 nicht in direkten Kontakt mit der Pumpe 4a tritt, sondern lediglich indirekt über einen Schlauch. Dies wird in der Konsole 10 dadurch erreicht, dass eine Rollenpumpe 4a angeordnet ist. Die Rollenpumpe 4a umfasst mehrere Rollen (nicht gezeigt), die bei einer Rotation der Rollenpumpe 4a einen Schlauch 15 (siehe Fig. 3) der Kassette 1 walken kann.

Hierdurch wird Irrigationsflüssigkeit durch den Schlauch 15 entlang des Irrigationsfluidweges 12 gepumpt, bis die Irrigationsflüssigkeit in die Patientenschnittstelle 5 geleitet werden kann.

Die Rollenpumpe 4a hat dabei sowohl eine Förderfunktion als auch eine Ventilfunktion. Dies wird dadurch erreicht, dass eine Bewegungseinrichtung 13 vorgesehen ist und dass die Rollenpumpe 4a in ihrer Position veränderbar ausgebildet ist. Die Bewegungseinrichtung 13 kann beispielsweise ein Elektromotor oder eine Drucklufteinrichtung sein. Mittels der Bewegungseinrichtung 13 kann die Rollenpumpe 4a an den Bogen 3 heran und/oder von dem Bogen 3 weg bewegt werden, in Figur 2 entlang des Pfeils 14a. Hierdurch wird eine unterschiedliche Anordnung der Pumpe 4a bzw. der Rollen der Pumpe 4a relativ zu der Kassette 1 ermöglicht. Insbesondere kann die Rollenpumpe 4a in zwei Positionen bewegt werden, eine erste Position, in der die Rollenpumpe 4a nah an dem Bogen 3 angeordnet ist und eine zweite Position, in der die Rollenpumpe 4a entfernt von dem Bogen 3 angeordnet ist. Diese beiden Positionen sind in Figur 3 gezeigt. Es ist ebenfalls denkbar, dass die Bewegungseinrichtung 13 die Rollen der Rollenpumpe 4a bewegt, zu dem Bogen 3 hin und/oder von dem Bogen 3 weg und so die zwei Positionen der Rollen realisiert.

In der ersten Position ist die Rollenpumpe 4a nah an dem Bogen 3 angeordnet. Hierdurch quetschen die Rollen (nicht gezeigt) der Rollenpumpe 4a den Schlauch 15 und verschließen ihn vollständig. Wenn die Rollenpumpe 4a still steht, wird auf diese Weise der Schlauch 15 verschlossen und es kann kein Irrigationsfluid durch den Irrigationsfluidweg 12 fließen. Hierdurch wird somit eine Ventilwirkung, insbesondere eine Schließwirkung durch die Rollenpumpe 4a erzeugt, da die Rollenpumpe 4a den Irrigationsfluidweg versperrt. Diese Ventilwirkung entspricht der Ventilwirkung des Sperrventils 6 gemäß Figur 1. Das heißt insbesondere, dass eine Bewegung der Rollenpumpe 4a den Irrigationsfluidweg öffnet und schließt, im Sinne eines Ventils.

In dieser ersten Position kann die Rollenpumpe 4a auch rotiert werden. In diesem Fall walken die Rollen der Rollenpumpe 4a den Schlauch 15 und drücken die in dem Schlauch 15 befindliche Irrigationsflüssigkeit in Richtung der Patientenschnittstelle 5. Hierdurch wird die Förderwirkung der Rollenpumpe 4a erzeugt.

Durch die Bewegungseinrichtung 13 kann die Rollenpumpe 4a in die zweite Position entfernt von dem Bogen 3 bewegt werden (siehe Figur 3). In diesem Fall wird der Schlauch 15 nicht durch die Rollenpumpe 4a verschlossen und Irrigationsfluid kann frei durch den Schlauch 15 fließen. Dies entspricht der Ventilwirkung des Sicherheitsventils 7 gemäß Figur 1.

Zusätzlich ist eine Federeinrichtung, hier in Form eines Federelements 16, vorgesehen, das auf die Rollenpumpe 4a wirkt. Das Federelement 16 kann in Form einer mechanischen Feder oder eines Druckluftzylinders ausgebildet sein. Das Federelement 16 erzeugt eine Rückstellkraft, die die Rollenpumpe 4a in Richtung der zweiten Position, also weg von dem Bogen 3, zieht. Hierdurch wird eine Sicherheitsfunktion gewährleistet. Im Betrieb ist die Rollenpumpe 4a gewöhnlich in der ersten Position und kann entweder Irrigationsfluid durch Rotation der Rollenpumpe 4a fördern oder bei Stillstehen der Rollenpumpe 4a den Fluidweg verschließen. Die Rückstellkraft des Federelements 16 wird dabei insbesondere durch die Bewegungseinrichtung 13 überwunden. Sollte es im Betrieb zu einem Stromausfall oder einem Defekt der Rollenpumpe 4a kommen, kann gewährleistet werden, dass dennoch Irrigationsfluid aus dem Irrigationsbehälter 11 zu der Patientenschnittstelle 5 fließen kann, da in diesem Fall die Rückstellkraft des Federelements 16 nicht mehr durch die Bewegungseinrichtung 13 überwunden wird und die Rollenpumpe 4a in die zweite Position gedrückt wird, wodurch der Irrigationsfluidweg 12 geöffnet wird.

Die Bewegungseinrichtung 13 kann insbesondere als ein Druckluftzylinder ausgebildet sein, der die Rückstellkraft des Federelements 16 überwinden kann.

Die Federeinrichtung 16 und/oder die Bewegungseinrichtung 13 erzeugt eine Kraft, die die Rollenpumpe 4a in Richtung der Kassette 1 und/oder die Kassette 1 in Richtung der Rollenpumpe 4a drückt. Die Federeinrichtung 16 sorgt hierdurch für einen Toleranzausgleich, wenn sich die Pumpe in der ersten Position befindet, da die Federeinrichtung einen definierten Anpressdruck zwischen Pumpe und Kassette ermöglicht, der unabhängig von den Toleranzen und somit dem Abstand zwischen Kassette Pumpe, ist. Der Toleranzausgleich kann ebenfalls durch die Bewegungseinrichtung 13 erfolgen, wenn die Bewegungseinrichtung 13 eine definierte Kraft erzeugen kann.

Es ist ebenfalls denkbar, dass die Federeinrichtung 16 durch eine Drucklufteinheit gebildet wird. Die Drucklufteinheit erzeugt einen Überdruck, der die Rollenpumpe 4a in die erste Position drückt. Bei einem Druckabfall wird die Rollenpumpe 4a in die zweite Position geschoben, da der Überdruck nicht ausreicht, um die Rollenpumpe 4a in der ersten Position zu halten.

Insofern kann der gesamte Irrigationsfluidweg frei von Ventilen ausgebildet werden, da die Rollenpumpe 4a sämtliche Ventilwirkungen übernimmt.

Nachdem das Irrigationsfluid in die Patientenschnittstelle 5 geströmt ist, kann es anschließend aspiriert werden. Dies wird durch eine zweite Rollenpumpe 4b erreicht, die in einem Aspirationsfluidweg 21 angeordnet ist. Die zweite Rollenpumpe 4b ist analog zu der ersten Rollenpumpe 4a an einem Bogen 3b angeordnet. Durch Rotation der zweiten Rollenpumpe 4b kann Aspirationsflüssigkeit aus der Patientenschnittstelle 5 abgesaugt werden und in einen Aspirationsbehälter 17 geleitet werden. Wenn die zweite Rollenpumpe 4b hingegen nicht rotiert wird, verschließt sie den Aspirationsfluidweg 21 und verhindert ein Strömen des Aspirationsfluids in dem Aspirationsfluidweg 21. Hierdurch übernimmt die Rollenpumpe 4b die Ventilwirkung des zweiten Sperrventils 6b gemäß Figur 1.

Entsprechend kann auch der Aspirationsfluidweg 21 frei von Ventilen ausgebildet sein.

Figur 3 zeigt die beiden Positionen der Rollenpumpe 4a.

Die Rollenpumpe 4a kann in zwei Positionen relativ zu dem Bogen 3 angeordnet sein. In der ersten Position (in Figur 3 rechts dargestellt) quetscht die Rollenpumpe 4a den Schlauch 15 durch die Rollen 23 und verhindert entweder, dass ein Fluid durch den Schlauch 15 strömt, oder kann Fluid durch den Schlauch 15 fördern, indem die Rollenpumpe 4a rotiert wird und so den Schlauch 15 gewalkt wird. Dabei treten die Rollen 23a der Rollenpumpe 4a nicht in direkten Kontakt mit der Flüssigkeit, da die Flüssigkeit ausschließlich durch den Schlauch 15 strömt. So wird eine Medientrennung zwischen Pumpe 4a und der Flüssigkeit erreicht. Es ist denkbar, dass andere Pumpen vorgesehen sind, die ebenfalls eine Medientrennung zwischen der Pumpe und dem Schlauch 15 erzeugen. Beispielsweise könnte ein Irrigationsbehälter durch eine Pumpeinrichtung auf verschiedene Höhen gefahren werden, sodass der hydrostatische Druck innerhalb des Schlauchsystems verändert wird und so eine Pumpfunktion erzielt wird.

Die zweite Position der Rollenpumpe 4a ist links dargestellt. In dieser Position wird der Schlauch 15 nicht gepresst und Fluid kann frei durch den Schlauch 15 fließen.

Figur 4 zeigt eine Kassette gemäß einer Ausführungsform der vorliegenden Erfindung.

Die Kassette 1 umfasst einen Bogen 3, über den eine Irrigationsfluidleitung 12 und eine Aspirationsfluidleitung 21 jeweils in Form eines Schlauchs 15 führen. In den Bogen 3 kann eine Rollenpumpe (in Figur 4 nicht gezeigt) eingesetzt werden, die die Aspirations- und Irrigationsleitung 21, 12 quetschen kann, um Fluid durch die Aspirations- und Irrigationsleitung 21, 12 zu pumpen, oder die Aspirations- und Irrigationsleitung 21, 12 zu verschließen.

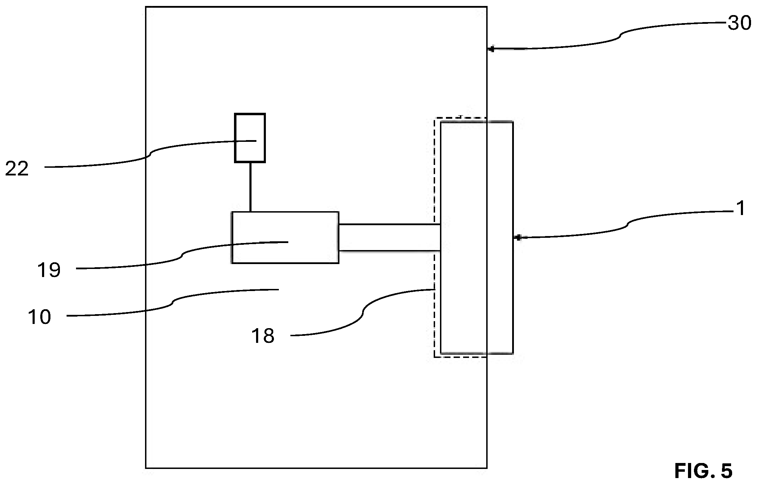

Figur 5 zeigt ein chirurgisches System gemäß einer Ausführungsform der vorliegenden Erfindung.

Das chirurgische System 30 umfasst eine Konsole 1 und eine Kassette 10, die in einem Schacht 18 der Konsole 1 eingesetzt ist. In der Konsole ist ein weiteres Federelement 19 vorgesehen, das in Form einer Feder, oder wie in Figur 5 gezeigt, in Form eines pneumatischen Kolbens ausgebildet sein kann. Das weitere Federelement 19 kann Teil der Federeinrichtung 16 sein (in Figur 5 nicht gezeigt). Insbesondere kann die Federeinrichtung 16 das weitere Federelement 19 umfassen.

Das weitere Federelement 19 ist dazu ausgebildet, die Kassette 10 in den Schacht 18 der Konsole 1 zu ziehen und dort zu fixieren, damit eine Rollenpumpe (nicht gezeigt) in die Kassette 10 eingreifen kann. Hierzu übt das Federelement 19 eine definierte Kraft auf die Kassette 10 aus. Hierdurch wird ein Toleranzausgleich zwischen Konsole 1 und Kassette 10 bewirkt, da die Kassette 10 nicht um einen spezifischen Weg in den Schacht 18 gezogen wird, sondern mit einer bestimmten Einzugskraft. Entsprechend ist der Anpressdruck der Rollenpumpe auf die Kassette 10, der von der Einzugskraft des weiteren Federelements 19 und der Anpresskraft der Rollenpumpe abhängig ist, im Wesentlichen konstant, sodass eine genaue Förderung des Fluids innerhalb der Kassette 10 ermöglicht werden kann.

In Figur 5 ist gezeigt, dass das weitere Federelement 19 die Kassette 1 in die Aufnahme der Kassette 1, hier in Form eines Schachts, ziehen kann. Somit ist die Rollenpumpe festgelegt, wohingegen die Kassette 1 beweglich ist. Auf diese Weise kann eine Toleranz der Rollenpumpe beziehungsweise der Kassette ausgeglichen werden. Bei einer weggeführten Positionierung von Rollenpumpe und Kassette kann es vorkommen, dass bei entsprechenden Toleranzen der Abstand zwischen Rollenpumpe und Kassette größer oder kleiner ist als gewünscht. Entsprechend ist der Anpressdruck zwischen Rollenpumpe und Kassette größer oder kleiner, wodurch ein unterschiedlicher Fluidstrom gefördert wird. Durch das weitere Federelement 19 wird dies ausgeglichen, da das weitere Federelement 19 die Kassette mit einer definierten Kraft auf die Rollenpumpe drückt. Der Toleranzausgleich wird somit durch die Kassette 1 erzielt. Es ist alternativ ebenfalls denkbar, dass das weitere Federelement 19 an der Rollenpumpe angeordnet ist und dazu ausgebildet ist, die Rollenpumpe auf die Kassette 1 zu drücken. In diesem Fall wäre die Position der Kassette 1 konstant und der Toleranzausgleich wird durch die Bewegung der Rollenpumpe bewirkt.

Das weitere Federelement ist als Druckluftzylinder ausgebildet. Die Einzugskraft des weiteren Federelements 19 kann durch die Veränderung des Luftdrucks des pneumatischen Elements eingestellt werden. Zusätzlich ist eine Steuerungseinheit 22 vorgesehen, die diese Einzugskraft aktiv steuert. Hierdurch kann die Einzugskraft auf die im Betrieb entstehenden Zustände angepasst werden. Beispielsweise kann die Einzugskraft erhöht werden, um die Verdrängung von Fluiden durch die Rollenpumpe an bestimmten Punkten zu minimieren.

Hinsichtlich weiterer vorteilhafter Ausgestaltungen der erfindungsgemäßen Vorrichtung wird zur Vermeidung von Wiederholungen auf den allgemeinen Teil der Beschreibung sowie auf die beigefügten Ansprüche verwiesen.

Schließlich sei ausdrücklich darauf hingewiesen, dass die voranstehend beschriebenen Ausführungsbeispiele der erfindungsgemäßen Vorrichtung lediglich zur Erörterung der beanspruchten Lehre dienen, diese jedoch nicht auf die Ausführungsbeispiele einschränken.

### Bezugszeichenliste

- 1: Kassette
- 2: Einlass
- 3: Bogen
- 4a, 4b: Rollenpumpe
- 5: Patientenschnittstelle
- 6: Sperrventil
- 7: Sicherheitsventil
- 8 9 10: Konsole
- 11: Irrigationsbehälter
- 12: Irrigationsfluidweg
- 13: Bewegungseinrichtung
- 14: Pfeil
- 15: Schlauch
- 16: Federelement
- 17: Aspirationsbehälter
- 18: Schacht
- 19: Weiteres Federelement
- 21: Aspirationsfluidsweg
- 22: Steuerungseinheit
- 23: Rollen

## Patentansprüche

1. Chirurgische Konsole (10) für ophthalmologische Eingriffe, umfassend ein Gehäuse mit darin befindlichen Funktionseinheiten, u.a. zur Aspiration und Irrigation, sowie mit Bedien- und Anzeigeelementen und Anschlüssen für ophthalmologische Geräte, wobei eine Aufnahme für eine ophthalmologische Kassette (1) vorgesehen ist, wobei zur Förderung von Flüssigkeit in der Kassette (1) mindestens eine Pumpe (4a. 4b) mit zumindest einem Pumpenelement (23) vorgesehen ist, wobei die Pumpe (4a. 4b) derart ausgebildet ist, dass eine Medientrennung zwischen der Pumpe (4a. 4b) und der Flüssigkeit in der Kassette (1) erfolgt, insbesondere wobei die Pumpe als eine über einen Rotor drehangetriebene Rollenpumpe (4a, 4b) ausgebildet ist, **dadurch gekennzeichnet, dass** eine Federeinrichtung (16) angeordnet ist, die dazu ausgebildet ist, die Kassette sowie die Pumpe und/oder das Pumpenelement mit einer definierten Kraft zueinander und/oder gegeneinander zu drücken.

2. Chirurgische Konsole (10) gemäß Anspruch 1, **dadurch gekennzeichnet, dass** eine Bewegungseinrichtung (13) vorgesehen ist, die dazu ausgebildet ist, die Anordnung der Pumpe (4a, 4b) und/oder eines Pumpenelements (23) sowie der Kassette relativ zueinander zu verändern, wobei die Pumpe (4a, 4b) und/oder das Pumpenelement (23) sowie die Kassette (1) in einer ersten Anordnung relativ zueinander anordenbar sind, in der die Pumpe (4a, 4b) ein Strömen der Flüssigkeit innerhalb der Kassette (1) verhindern kann und wobei die Pumpe (4a, 4b) und/oder das Pumpenelement (23) sowie die Kassette (1) in einer zweiten Anordnung relativ zueinander anordenbar sind, in der die Pumpe (4a, 4b) ein Strömen der Flüssigkeit innerhalb der Kassette (1) ermöglicht.

3. Chirurgische Konsole (10) gemäß Anspruch 2, **dadurch gekennzeichnet, dass** die Federeinrichtung (16) eine Feder und/oder ein pneumatisches Element umfasst.

4. Chirurgische Konsole (10) gemäß einem der Ansprüche 1 bis 3, **dadurch gekennzeichnet, dass** ein Behälter (11) mit Irrigationsflüssigkeit vorgesehen ist, wobei ein Fluidweg (12) zwischen dem Behälter (11) mit Irrigationsflüssigkeit und den Anschlüssen (5) für die ophthalmischen Geräte frei von Ventilen ausgebildet ist.

5. Chirurgische Konsole (10) gemäß einem der Ansprüche 1 bis 4, **dadurch gekennzeichnet, dass** ein Behälter (17) zur Aufnahme von Aspirationsflüssigkeit vorgesehen ist, wobei ein Fluidweg (21) zwischen dem Behälter (17) für Aspirationsflüssigkeit und den Anschlüssen (5) für die ophthalmischen Geräte frei von Ventilen ausgebildet ist.

6. Chirurgische Konsole (10) gemäß einem der Ansprüche 1 bis 5, **dadurch gekennzeichnet, dass** eine weitere Federeinrichtung (19) angeordnet ist, wobei die weitere Federeinrichtung (19) dazu ausgebildet ist, die Kassette (1) mit einer definierten Einzugskraft gegen die Pumpe zu drücken.

7. Chirurgische Konsole (10) gemäß Anspruch 6, **dadurch gekennzeichnet, dass** die weitere Federeinrichtung (19) ein pneumatisches Element umfasst, wobei das pneumatische Element dazu ausgebildet ist, die Einzugskraft zu steuern.

8. Chirurgische Konsole (10) gemäß einem der Ansprüche 1 bis 7, **dadurch gekennzeichnet, dass** durch die Pumpe (4a, 4b) eine Flüssigkeit in der Kassette (1) förderbar ist, wenn sich die Pumpe (4a, 4b) in der ersten Position befindet.

9. Chirurgisches System (30) umfassend eine chirurgische Konsole (10) gemäß einem der Ansprüche 1 bis 8 sowie eine ophthalmologische Kassette (1), die in der Aufnahme (18) der chirurgischen Konsole (10) vorgesehen ist, wobei die Kassette (1) dazu ausgebildet ist, mit der Pumpe (4a, 4b) der chirurgischen Konsole (10) in Wirkverbindung zu treten, wenn die Kassette (1) in der Aufnahme (18) angeordnet ist.

10. Chirurgisches System (30) gemäß Anspruch 9, **dadurch gekennzeichnet, dass** ein Fluidweg innerhalb der Kassette (1) durch die Pumpe (4a, 4b) verschließbar ist, wenn die Pumpe (4a, 4b) in der ersten Position angeordnet ist, wobei die Pumpe (4a, 4b) eine Flüssigkeit in dem Fluidweg fördern kann, wenn die Pumpe (4a, 4b) in der ersten Position angeordnet ist und wobei eine Flüssigkeit durch den Fluidweg strömen kann, wenn die Pumpe (4a, 4b) in der zweiten Position angeordnet ist.
